# EUROPEAN PATENT SPECIFICATION

(11) **EP 4 203 923 B1**
(45) Date of publication and mention of the grant of the patent: **01.10.2025**
(21) Application number: 21820122.6
(22) Date of filing: 23.08.2021
(51) Int. Cl.: A61K 9/28, A61K 31/519, A61P 35/00, A61P 35/02, A61K 9/20

(54) **PHARMACEUTICAL COMPOSITION COMPRISING IBRUTINIB**
PHARMAZEUTISCHE ZUSAMMENSETZUNG MIT IBRUTINIB
COMPOSITION PHARMACEUTIQUE COMPRENANT DE L'IBRUTINIB

(30) Priority: 28.08.2020 EP 20193347
(43) Date of publication of application: 05.07.2023
(73) Proprietor: Synthon B.V., 6545 CM Nijmegen (NL)
(72) Inventor: VIVANCOS MARTINEZ, Marta, 08830 Sant Boi de Llobregat (ES); ALVAREZ FERNANDEZ, Lisardo, 08830 Sant Boi de Llobregat (ES); KUMAR, Rohit, 08830 Sant Boi de Llobregat (ES)
(74) Representative: Mendivil Gil, Maria Dolores
(86) International application number: PCT/EP2021/073253
(87) International publication number: WO 2022/043251

(56) References cited:
- EP-A1- 3 501 609
- WO-A1-2013/184572
- WO-A1-2020/127912
- WO-A2-2018/033941

## Description

### BACKGROUND OF THE PRESENT INVENTION

Ibrutinib, chemically 1-[(3*R*)-3-[4-amino-3-(4-phenoxyphenyl)-1*H*-pyrazolo[3,4-*d*]pyrimidin-1-yl]piperidin-1-yl]prop-2-en-1-one of formula (I), 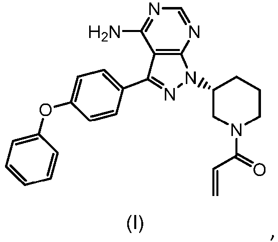 is a pharmaceutically active compound. It is used for the treatment of chronic lymphocytic leukaemia (CLL) in previously untreated patients and in patients who have received at least one previous treatment. It is also used for the treatment of mantle cell lymphoma (MCL) in patients whose disease does not respond to or has come back after previous treatment and to treat Waldenström's macroglobulinaemia (WM), also known as lymphoplasmacytic lymphoma, in patients who have had previous treatment or who cannot have chemo immunotherapy.

Ibrutinib is marketed by Janssen/Pharmacyclics under the brand name Imbruvica^{®} and is disclosed in WO2008039218. Imbruvica^{®} was initially only supplied as hard capsule in one strength: 140 mg. Patients take either 3 or 4 capsules once daily, depending on the disease to be treated. In 2018 FDA approved a new tablet formulation of Imbruvica^{®}. The tablet is available in the strengths 140, 280, 420 and 560 mg and thus reduces pill burden for patients.

Higher strengths derive in big tablets that can be not easily swallowed. Uncoated tablets are rougher, may be more difficult to swallow, and often leave a bad taste in the mouth when swallowed. A coated tablet generally goes down easier and with less aftertaste. Therefore, a film coating tablet is preferred to facilitate its swallowing and improve patient acceptability.

Several crystalline forms of ibrutinib are described in literature. WO2013184572 discloses crystalline forms A, B and C and solvated forms D (methyl isobutyl ketone solvate), E (toluene solvate) and F (methanol solvate). The marketed product Imbruvica^{®}, both capsule and tablet formulation, contains crystalline ibrutinib form A. Ibrutinib from A is a very stable compound, but being a BCS class II compound, it exhibits low aqueous solubility which affects dissolution behavior. Crystalline form C of ibrutinib is less stable than ibrutinib form A, but exhibits a significantly higher solubility.

Pharmaceutical compositions comprising ibrutinib in crystalline form C are known from prior art. EP3501609 and WO2020/127912 disclose compositions in the form of capsules and tablets (respectively) comprising ibrutinib form C. Both documents describe the compositions, which are free of surfactants. While both capsule and tablet composition of Imbruvica^{®} contains a considerable amount of the anionic surfactant sodium lauryl sulphate (SLS), it is desirable to avoid having such compounds in the composition. It is well known that their presence may give rise to irritation of the gastrointestinal tract. In addition, the presence of SLS has been found to have a negative influence on stability of form C of ibrutinib as well as its solubility and thus bioavailability.

However, the problem arises when the tablet formulation comprising ibrutinib form C is further coated. It was found by the inventors that a common film coating composition promotes polymorphic conversion from Ibrutinib Form C to Form A. Film coating is characterized by application of an excipient suspension formulation, consisting of polymer (film former), plasticizer, colouring/opacifying agent, and solvent, directly onto the tablet. The inventors have found surprisingly that the use of a coating composition that does not contain plasticizer solves this issue.

### BRIEF DESCRIPTION OF THE PRESENT INVENTION

The present invention provides a stable film coated tablet composition comprising ibrutinib and one or more pharmaceutically acceptable excipients, characterized in that:
- Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation;
   ·The composition is free of surfactant, and
- The coating is free of plasticizer,
characterized in that the coating comprises hydroxypropylmethylcellulose and hydroxypropylcellulose as film formers.

It also provides said film coated tablet composition for use as a medicament. Also disclosed, but not claimed, is a process for preparing the tablet composition comprising a granulation step.

Said pharmaceutical composition may be used as a medicament, particularly in the treatment of chronic lymphocytic leukaemia (CLL), mantle cell lymphoma (MCL), Waldenström's macroglobulinaemia (WM) and chronic graft-versus-host disease (cGVHD).

### BRIEF DESCRIPTION OF THE DRAWINGS

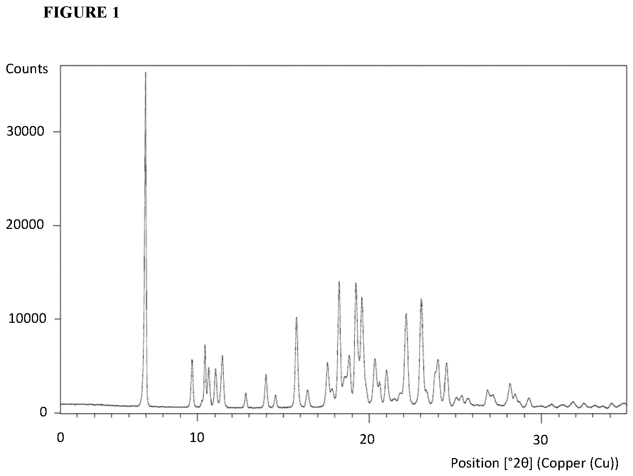

Figure 1 shows the full XRPD pattern of ibrutinib form C. For measurement conditions see the Examples section.

### DETAILED DESCRIPTION OF THE PRESENT INVENTION

A tablet composition exhibiting a similar dissolution profile as Imbruvica^{®} can be obtained by using ibrutinib form C. Such composition has an advantage that it does not require the use of surfactant. However, higher strengths of ibrutinib derive in big size tablets. Uncoated tablets are rougher, may be more difficult to swallow, and often leave a bad taste in the mouth when swallowed. A coated tablet generally goes down easier and with less aftertaste. A film coating tablet is preferred to facilitate its swallowing and improve patient acceptability. Film coating is characterized by application of an excipient suspension formulation, consisting of polymer (film former), plasticizer, colouring/opacifying agent, and solvent, directly onto the tablet. However, a common film coating compositions promote polymorphic conversion from Ibrutinib Form C to Form A. It was surprisingly found out that the use of a coating composition that does not contain plasticizer, but is composed by hydroxypropylmethylcellulose (HPMC) and hydroxypropylcellulose (HPC) as film formers resulted in coated tablets that are easy to swallow and stable.

The tablet composition of the present invention is very stable and even after storage at elevated temperature or increased relative humidity, ibrutinib form C in the composition does not convert into ibrutinib form A or any other crystalline form of ibrutinib. The tablet is prepared by a robust and cost effective process and is bioequivalent to Imbruvica^{®}.

The present invention thus provides a coated tablet composition comprising ibrutinib and one or more pharmaceutically acceptable excipients, characterized in that:
- Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation;
   · The composition is free of surfactant, and
- The coating is free of plasticizer,
characterized in that the coating comprises hydroxypropylmethylcellulose and hydroxypropylcellulose as film formers.

The XRPD pattern of ibrutinib form C may further comprise characteristic peaks at the following 2 theta (± 0.2) angles: 15.7°, 17.5°, 20.3°, 22.1° and 24.0°, measured using a Cu Kα radiation. The XRPD pattern of ibrutinib form C is shown in figure 1. Ibrutinib form C is present in the tablet composition of the present invention in an amount from 60 to 80% w/w relative to the total weight of the tablet. More preferably, ibrutinib form C is present in the tablet composition in an amount from 65 to 75% w/w relative to the total weight of the tablet.

The tablets of the present invention are coated by a film coat. The coating material has no influence on the release rate, except of an inherent short initial delay in dissolution due to the time necessary to dissolve the coat. The coating composition used does not contain plasticizer. Plasticizers are molecules with shorter chain lengths than the film forming polymers that embed themselves between the polymer chains, thereby increasing the free volume of these polymer chains, lowering the glass transition temperature/melting point of these film-forming polymers and increasing their flexibility. Common molecules used as plasticizers are (oligomers of) ethylene glycol or propylene glycol, dialkylphtalate esters, trialkylcitrates and acetyl trialkylcitrates, and glycerol and its mono-, di- and triesters. Typical examples of molecules for use as plasticizer in film coatings for pharmaceutical tablets are PEG 400 (polyethylene glycol oligomer), GMCC (mixture of glycerol monoesters with caprylate and caprate) and triacetin (glycerol trimester with acetic acid).

It was found that after film-coating with coating compositions comprising plasticizers, ibrutinib converts from polymorphic from C to more stable polymorphic form A.

The tablets according to this invention are coated with a coating composed by hydroxypropylmethylcellulose (HPMC) and hydroxypropylcellulose (HPC) as film formers and optionally colouring/opacifying agents. Preferably, the amount of hydroxpropylcellulose in the film coating composition is between 30 and 40 weight%, preferably between 33 and 37 weight% relative to the total weight of the film coating composition. Preferably the film coating comprises: 33-37% hydroxypropylcellulose, 30-36% hydroxypropylmethylcellulose, 25-30% titanium dioxide and 2-5% iron oxides, all defined as weight relative to the total film coating weight. The example of commercially available film-coating without plasticizer is Opadry^{®} I 20A23676 yellow. Opadry^{®} I 20A23676 yellow is a mixture of 35.0 % (w/w) hydroxypropylcellulose (HPC), 34.0% (w/w) hydroxypropylmethylcellulose (HPMC), 27.55% (w/w) titanium dioxide and 3.45% (w/w) iron oxides.

The coating may be performed by applying the film forming polymers, with or without other pharmaceutically inert excipients, as a solution/suspension. Coating is done using any conventional coating technique known in the art, such as spray coating in a conventional coating pan or fluidized bed processor; or dip coating.

The tablet compositions according to the present invention comprise, besides ibrutinib form C, one or more pharmaceutically acceptable excipients. The excipients to be used in accordance with the present invention are well-known and are those excipients which are conventionally used by the person skilled in the art. The pharmaceutically acceptable excipients are chosen from one or more diluents, binders, disintegrants, glidants or lubricants.

The pharmaceutical composition according to the present invention comprises preferably 10-30% w/w of one or more diluents, 0-7% w/w of one or more binders, 2-15% w/w of one or more disintegrants, 0.25-1.0% w/w of one or more glidants and 0.25-2.0% w/w of one or more lubricants, all relative to the total tablet weight.

The diluent to be used in accordance with the present invention may be any diluent known to a person of ordinary skill in the art. Particularly, the diluent to be used in accordance with the present invention is an inorganic diluent, polysaccharide, mono- or disaccharide or sugar alcohol. Lactose and microcrystalline cellulose are particularly preferred diluents.

In one embodiment of the present invention, the diluent is added as intragranular component. In another embodiment, the diluent is added partially to the intragranular phase and partially to the extragranular phase.

The diluent to be used in accordance with the present invention may contain lactose, microcrystalline cellulose, a basifying excipient or mixtures thereof.

The binder to be used in accordance with the present invention may be any binder known to a person of ordinary skill in the art. Suitable binders are selected from the group consisting of sodium carboxymethylcellulose, polyvinyl pyrrolidone (PVP), copovidone, polyvinyl pyrrolidone-vinyl acetate (PVP/VA) copolymer, hydroxypropylcellulose (HPC), hydroxypropylmethylcellulose (HPMC) or ethyl cellulose. PVP, copovidone and HPMC are particularly preferred binders.

The binder is added to the intragranular phase.

The disintegrant to be used in accordance with the present invention may be any disintegrant known to a person of ordinary skill in the art. Suitable disintegrants to be used in accordance with the present invention are selected from the group consisting of croscarmellose sodium, crospovidone, low-substituted hydroxypropylcellulose (HPC) or sodium starch glycolate. Croscarmellose sodium is a particularly preferred disintegrant.

In one embodiment of the present invention, the disintegrant is added as to the intragranular phase. In another embodiment, the disintegrant is added partially to the intragranular phase and partially to the extragranular phase.

The glidant to be used in accordance with the present invention may be any glidant known to a person of ordinary skill in the art. Colloidal silicon dioxide is a particularly preferred glidant.

In one embodiment of the present invention, the glidant is added as intragranular component. In another embodiment, the glidant is added to the extragranular phase.

The lubricant to be used in accordance with the present invention may be any lubricant known to a person of ordinary skill in the art. Magnesium stearate is a particularly preferred lubricant.

The lubricant may be added as extragranular component or it may be divided over the intragranular and extragranular phase. In a preferred embodiment, the lubricant is added partially to the intragranular phase and partially to the extragranular phase.

The tablet composition in accordance with the present invention is free of substance that works as surfactant. Surfactants are amphiphilic molecules that contain both hydrophilic and lipophilic groups. Surfactants may have several uses in pharmaceuticals, e.g. to solubilize hydrophobic drugs in aqueous media or to improve drug absorption and penetration. Surfactants are classified into ionic surfactants and non-ionic surfactants. Ionic surfactants are sub classified into anionic surfactants where the hydrophilic group dissociates into anions in aqueous solutions, cationic surfactants that dissociate into cations and amphoteric surfactants that dissociate in anions and cations often depending on pH. Examples of non-ionic surfactants are polyol esters including glycol and glycerol esters and sorbitan derivatives, polyoxyethylene esters including polyethylene glycol and poloxamers, which are non-ionic triblock copolymers composed of polyoxypropylene flanked by polyoxyethylene. Examples of ionic surfactants are sodium lauryl sulfate, docusate, alkyl ether phosphates and quartenary ammonium salts. The hydrophile-lipophile balance (HLB) number is used as a measure of the ratio of the hydrophilic and lipophilic groups in the surfactant and defines the affinity for water or oil. HLB numbers above 10 indicate an affinity for water (hydrophilic) and below 10 an affinity for oil (lipophilic).

The tablet composition of the present composition is free of surfactant and does thus not contain SLS or any other surfactant.

The tablet composition according to the present invention is packaged in primary packaging material, e.g. blisters and bottles. The tablet composition of the present invention is preferably packaged in capped bottles. HDPE bottles are particularly preferred. The capped bottles may comprise means to absorb water by having a cap containing desiccant, e.g. silica gel.

The pharmaceutical composition of the present invention exhibits excellent long term stability. Moreover, the pharmaceutical composition of the present invention is very suitable for production on commercial scale making use of equipment and techniques commonly used in industry.

Disclosed, but not claimed, is a process to prepare a tablet composition comprising ibrutinib form C and one or more pharmaceutically acceptable excipients comprising a granulation step. The granulation processes applied are simple and cost effective and include a standard wet or dry granulation technique.

The wet granulation process is performed with a granulation solvent selected from the group consisting of water, acetone, ethanol, isopropanol or a mixture thereof.

Preferably, the process to prepare the tablet composition of the present invention comprises a dry granulation step. The dry granulation process is conducted by either slugging or roller compaction. The advantage of the dry granulation over the process of wet granulation is that it does not use any organic solvents or water. The risk of stability issues is minimized in this way, especially when active pharmaceutical ingredients are used that are prone to (polymorphic) conversion. However, due to the relative high amount of API in the tablet composition of the present invention, applying the process of dry granulation is not the obvious choice.

The tablet prepared by applying the step of dry granulation comprises, besides ibrutinib form C, one or more pharmaceutically acceptable binders, diluents, disintegrants, glidants or lubricants. Preferably, the tablet prepared by using the step of dry granulation comprises ibrutinib form C, lactose, microcrystalline cellulose, polyvinylpyrrolidone (PVP), croscarmellose sodium, silicon dioxide and magnesium stearate. Optionally, HPMC may be present as well.

The tablet composition in accordance with the present invention may be used as a medicament. The composition typically may be used in the treatment of chronic lymphocytic leukaemia (CLL), mantle cell lymphoma (MCL), Waldenström's macroglobulinaemia (WM) and chronic graft-versus-host disease (cGVHD).
The following examples are intended to illustrate the scope of the present invention but not to limit it thereto.

### EXAMPLES

The full XRPD pattern of ibrutinib form C of Figure 1 was obtained using a Bruker-AXS D8 Vario diffractometer with θ/2θ geometry (reflection mode), equipped with a Lynxeye detector and applying the following measurement conditions:
Start angle (2θ): 2.0°
End angle (2θ): 35.0°
Scan step width: 0.02°
Scan step time: between 0.2-2.0 seconds
Radiation type: Cu
Radiation wavelengths: 1.5406Å (Kα1), primary monochromator used
Exit slit: 6.0 mm
Focus slit: 0.2 mm
Divergence slit: Variable (V20)
Antiscatter slit: 11.8 mm
Receiving slit: 20.7 mm

### Reference example 1: uncoated tablets comprising ibrutinib form C and no surfactant

The uncoated tablets comprising ibrutinib form C were prepared by the process of dry granulation and have the composition as given in table 1.

**Table 1**

| **Component** | Tablet composition | |
|---|---|---|
| | % | mg/tab |
| *Intragranular components* | | |
| Ibrutinib form C | 70.0% | 560.0 |
| Lactose monohydrate (SUPERTAB 11SD) | 14.0% | 112.0 |
| Povidone K25 (KOLLIDON K25) | 2.0% | 16.0 |
| Croscarmellose sodium (AC-DI-SOL) | 5.0% | 40.0 |
| Magnesium stearate (MF-2-V) | 0.5% | 4.0 |

| *Extragranular components* | | |
|---|---|---|
| Colloidal silicon dioxide (Aerosil 200 VV Pharma) | 0.5% | 4.0 |
| Croscarmellose sodium (AC-DI-SOL) | 2.0% | 16.0 |
| Microcrystalline cellulose (VIVAPUR 302) | 5.5% | 44.0 |
| Magnesium stearate (MF-2-V) | 0.5% | 4.0 |
| **UNCOATED TABLET WEIGHT** | **100.00%** | **800.000** |

The tablets were prepared by using the process of dry granulation. The granulate was mixed with the extragranular components and compressed using a rotating tablet press using appropriate punches.

### Example 1: coating compatibility study

A compatibility study using binary mixture of ibrutinib with different coating compositions was performed. Only the coating composition comprising HPMC and HPC is according to the claimed invention; the other coating compositions are reference compositions.

Film coating-drug substance ratio was established according to the relative estimated amounts of the components in the formulation. In order to promote the interaction of the drug substance with the corresponding film coating, the relative amount of the excipients in the formulation was increased ten times in relation to the drug substance. Samples were stored at 40°C/75 % RH in open containers for 1 month. Polymorphism was checked by XRPD analyses. The results as given in table 2.

**Table 2**

| **Binary mixtures** | **T=0** | **T=1 month at 40°C_75%RH** |
|---|---|---|
| | | **Open dish** |
| ITN: Coating composition: | ITN: Form C | ITN: Form A |
| Polymer: PVA | | |
| Plasticizer: PEG | | |
| Detackifier: Talc | | |
| Opacifier: TiO2 | | |
| Colorant: iron oxides | | |
| (Opadry^{®} II yellow 85F32004) | | |
| ITN: Coating composition: | ITN: Form C | ITN: Form C+A |
| Polymer: HPMC | | |
| Plasticizer: PEG | | |
| Detackifier: Talc | | |
| Opacifier: TiO2 | | |
| Colorant: iron oxides | | |
| (Opadry^{®} yellow 03F220119) | | |
| ITN: Coating composition: | ITN: Form C | ITN: Form A |
| Polymer: HPMC | | |
| Plasticizer: Triacetin | | |
| Opacifier: TiO2 | | |
| Colorant: iron oxides | | |
| (Opadry^{®} II yellow 32K220025) | | |
| ITN: Coating composition: | ITN: Form C | ITN: Form C |
| Polymer: HPMC/HPC | | |
| Opacifier: TiO2 | | |
| Colorant: iron oxides | | |
| (Opadry^{®} I 20A23676 yellow) | | |
| ITN: Coating composition: | ITN: Form C | ITN: Form A |
| Polymer: PVA-PEG | | |
| Plasticizer: GMCC | | |
| Colorant: iron oxides | | |
| (Opadry^{®} QX RED 321A250023) | | |

Polymorphic conversion is observed in all the binary mixtures stored at 40°C/75%RH in open dish except in the binary mixture of Ibrutinib with a coating composition without plasticizer (Opadry^{®} I 20A23676 yellow).

### Example 2

Tablets of reference example 1 were coated with 24 mg per tablet of coating composition comprising plasticizer (Opadry^{®} II yellow 85F32004) (not according to the claimed invention) and with 24 mg per tablet of coating composition without plasticizer (Opadry^{®} I 20A23676 yellow). Two formulations of coated tablets and uncoated tablets of reference example 1 were packaged in high protective packaging (Aluminium/Aluminium blister) and stored at 40°C/75%HR for 3 months.

The results are given in table 3.

**Table 3**

| **Conditions: 40°C/75%RH** | **T=0** | **T=2 months** | **T=3months** |
|---|---|---|---|
| **Packaging: Al/Al blister** | | | |
| Uncoated tablets | Form C | Form C | Form C |
| Tablets coated with Opadry^{®} II yellow 85F32004 | Form C | Form A+C | Form A+C |
| Tablets coated with Opadry^{®} I 20A23676 yellow | Form C | Form C | Form C |

Polymorphic conversion from form C to form A was observed in formulation coated with coating comprising plasticizer (Opadry^{®} II yellow 85F32004) after 2 months in accelerated conditions (40°C/75%RH) even though the tablets were protected with a high protecting packaging.

## Claims

1. A film coated tablet composition comprising ibrutinib and one or more pharmaceutically acceptable excipients, wherein:
• Ibrutinib is form C, having characteristic peaks in the X-ray powder diffraction pattern at the following 2 theta (± 0.2) angles: 6.9°, 18.2°, 19.2°, 19.6° and 23.0°, measured using a Cu Kα radiation;
• The composition is free of surfactant, and
• The coating is free of plasticizer,
**characterized in that** the coating comprises hydroxypropylmethylcellulose and hydroxypropylcellulose as film formers.

2. The film coated tablet composition according to claim 1, wherein the amount of hydroxypropylcellulose in the film coating is 30-40 weight%, preferably 33-37 weight% relative to the weight of the film coating.

3. The film coated tablet composition according to claim 1, wherein the amount of hydroxypropylmethylcellulose in the film coating is 27-39% weight%, preferably 30-36 weight% relative to the weight of the film coating.

4. The film coated tablet composition according to any of claims 1 to 3, wherein the film coating comprises: 33-37% hydroxypropylcellulose, 30-36% hydroxypropylmethylcellulose, 25-30% titanium dioxide and 2-5% iron oxides, all defined as weight relative to the total film coating weight.

5. The film coated tablet composition according to any of the previous claims, wherein the pharmaceutically acceptable excipients are chosen from one or more diluents, binders, disintegrants, glidants or lubricants.

6. The film coated tablet composition according to any one of the previous claims for use as a medicament.

7. The film coated tablet composition according to claim 6 for use in the treatment of chronic lymphocytic leukaemia (CLL), mantle cell lymphoma (MCL), Waldenström's macroglobulinaemia (WM) and chronic graft-versus-host disease (cGVHD).

## Patentansprüche

1. Filmbeschichtete Tablettenzusammensetzung, die Ibrutinib und einen oder mehrere pharmazeutisch verträgliche Hilfsstoffe umfasst, wobei:
• Ibrutinib Form C ist, mit charakteristischen Signalspitzen im Röntgenpulverdiffraktionsmuster bei den folgenden 2-Theta (± 0,2)-Winkeln: 6,9°, 18,2°, 19,2°, 19,6° und 23,0°, gemessen unter Verwendung einer Cu-Kα-Strahlung;
• die Zusammensetzung frei von oberflächenaktiven Mitteln ist, und
• die Beschichtung frei von Weichmachern ist,
**dadurch gekennzeichnet, dass** die Beschichtung Hydroxypropylmethylcellulose und Hydroxypropylcellulose als Filmbildner umfasst.

2. Filmbeschichtete Tablettenzusammensetzung nach Anspruch 1, wobei die Menge an Hydroxypropylcellulose in der Filmbeschichtung 30-40 Gew.-%, bevorzugt 33-37 Gew.-% relativ zum Gewicht der Filmbeschichtung beträgt.

3. Filmbeschichtete Tablettenzusammensetzung nach Anspruch 1, wobei die Menge an Hydroxypropylmethylcellulose in der Filmbeschichtung 27-39 Gew.-%, bevorzugt 30-36 Gew.-% relativ zum Gewicht der Filmbeschichtung beträgt.

4. Filmbeschichtete Tablettenzusammensetzung nach einem beliebigen der Ansprüche 1 bis 3, wobei die Filmbeschichtung umfasst: 33-37% Hydroxypropylcellulose, 30-36% Hydroxypropylmethylcellulose, 25-30% Titandioxid und 2-5% Eisenoxide, alle definiert als Gewicht relativ zum gesamten Filmbeschichtungsgewicht.

5. Filmbeschichtete Tablettenzusammensetzung nach einem beliebigen der voranstehenden Ansprüche, wobei die pharmazeutisch verträglichen Hilfsstoffe aus einem oder mehreren Verdünnungsmitteln, Bindemitteln, Sprengmitteln, Gleitmitteln oder Schmiermitteln ausgewählt sind.

6. Filmbeschichtete Tablettenzusammensetzung nach einem beliebigen der voranstehenden Ansprüche zur Verwendung als Arzneimittel.

7. Filmbeschichtete Tablettenzusammensetzung nach Anspruch 6 zur Verwendung bei der Behandlung von chronischer lymphatischer Leukämie (CLL), Mantelzell-Lymphom (*mantle cell lymphoma,* MCL), Waldenström-Makroglobulinämie (WM) und chronischer Transplantat-gegen-Wirt-Reaktion (*chronic graft-versus-host disease,* cGVHD).

## Revendications

1. Une composition de comprimé pelliculé comprenant de l'ibrutinib et un ou plusieurs excipients pharmaceutiquement acceptables, dans laquelle :
• l'ibrutinib est de forme C, ayant des pics caractéristiques dans le diagramme de diffraction des rayons X sur poudre aux angles 2 thêta (± 0,2) suivants : 6,9°, 18,2° 19,2°, 19,6° et 23,0° mesurés à l'aide d'un rayonnement Cu Kα ;
• la composition est exempte de tensioactif, et
• l'enrobage est exempt de plastifiant
**caractérisé en ce que** le revêtement comprend de l'hydroxypropylméthylcellulose et de l'hydroxypropylcellulose comme agents filmogènes.

2. La composition de comprimé pelliculé selon la revendication 1, dans laquelle la quantité d'hydroxypropylcellulose dans le pelliculage est de 30 à 40 % en poids, de préférence de 33 à 37 % en poids par rapport au poids du pelliculage.

3. La composition de comprimé pelliculé selon la revendication 1, dans laquelle la quantité d'hydroxypropylméthylcellulose dans le pelliculage est de 27 à 39 % en poids, de préférence de 30 à 36 % en poids par rapport au poids du pelliculage.

4. La composition de comprimé pelliculé selon l'une quelconque des revendications 1 à 3, dans laquelle le pelliculage comprend : de 33 à 37 % d'hydroxypropylcellulose, de 30 à 36 % d'hydroxypropylméthylcellulose, de 25 à 30 % de dioxyde de titane et de 2 à 5 % d'oxydes de fer, tous définis en poids par rapport au poids total du pelliculage.

5. La composition de comprimé pelliculé selon l'une quelconque des revendications précédentes, dans laquelle les excipients pharmaceutiquement acceptables sont choisis parmi un ou plusieurs diluants, liants, désintégrants, agents de glissement ou lubrifiants.

6. La composition de comprimé pelliculé selon l'une quelconque des revendications précédentes pour une utilisation comme médicament.

7. La composition de comprimé pelliculé selon la revendication 6 pour une utilisation dans le traitement de la leucémie lymphoïde chronique (LLC), du lymphome à cellules du manteau (LCM), de la macroglobulinémie de Waldenström (MW) et de la maladie du greffon contre l'hôte chronique (GvHDc).
